# EUROPEAN PATENT SPECIFICATION

(11) **EP 3 195 293 B1**
(45) Date of publication and mention of the grant of the patent: **31.10.2018**
(21) Application number: 14809970.8
(22) Date of filing: 15.09.2014
(51) Int. Cl.: G09B 23/28, G09B 23/30

(54) **THREE-DIMENSIONAL MODEL AND METHOD FOR TRAINING PITUITARY GLAND SURGERY AND METHOD FOR EVALUATING THE RESULT THEREOF**
DREIDIMENSIONALES MODELL UND VERFAHREN ZUM TRAINIEREN VON CHIRURGISCHEN EINGRIFFEN DER HIRNANHANGSDRÜSE UND VERFAHREN ZUR BEURTEILUNG DES ERGEBNISSES DAVON
MODÈLE TRIDIMENSIONNEL ET MÉTHODE D'APPRENTISSAGE D'UNE INTERVENTION CHIRURGICALE DE L'HYPOPHYSE AINSI QUE MÉTHODE D'ÉVALUATION DE SON RÉSULTAT

(43) Date of publication of application: 26.07.2017
(73) Proprietor: Kantonsspital St. Gallen, 9007 St. Gallen (CH)
(72) Inventor: FOURNIER, Jean-Yves, CH-9007 St. Gallen (CH); TASMAN, Abel-Jan, CH-9007 St. Gallen (CH)
(74) Representative: Patentanwälte Bregenzer und Reule Partnerschaftsgesellschaft mbB
(86) International application number: PCT/IB2014/001826
(87) International publication number: WO 2016/042350

(56) References cited:
- TAKESHI OKUDA ET AL: "Training in endoscopic endonasal transsphenoidal surgery using a skull model and eggs", ACTA NEUROCHIRURGICA ; THE EUROPEAN JOURNAL OF NEUROSURGERY, SPRINGER-VERLAG, VI, vol. 152, no. 10, 11 August 2010 (2010-08-11), pages 1801-1804, XP019853144, ISSN: 0942-0940
- TAKESHI OKUDA ET AL: "The chicken egg and skull model of endoscopic endonasal transsphenoidal surgery improves trainee drilling skills", ACTA NEUROCHIRURGICA, vol. 156, no. 7, 8 March 2014 (2014-03-08) , pages 1403-1407, XP055187520, ISSN: 0001-6268, DOI: 10.1007/s00701-014-2035-7

## Description

The invention relates to a three-dimensional model as defined in the preamble of claim 1 and a method for training pituitary gland surgery as well as a method for evaluating the result of such training. Such three-dimensional model is e.g. illustrated in TAKESHI OKUDA ET AL, "Training in endoscopic endonasal transsphenoidal surgery using a skull model and eggs", ACTA NEUROCHIRURGICA ; THE EUROPEAN JOURNAL OF NEUROSURGERY, SPRINGER-VERLAG, VI, (20100811), vol. 152, no. 10, ISSN 0942-0940, pages 1801 - 1804. A method using such model is disclosed in TAKESHI OKUDA ET AL, "The chicken egg and skull model of endoscopic endonasal transsphenoidal surgery improves trainee drilling skills", ACTA NEUROCHIRURGICA, (20140308), vol. 156, no. 7, doi:10.1007/s00701-014-2035-7, ISSN 0001-6268, pages 1403 - 1407.

Transsphenoidal endoscopic surgery has gained popularity in the last two decades and is becoming the standard technique for resection of pituitary tumors/adenomas. Neurosurgical residents have practically no endoscopic experience when they reach the stadium of transsphenoidal procedures. Cadaveric workshops are expensive and cannot be repeated very frequently. Endoscopic software simulators do exist but are very expensive as well and although they allow repetition they lack the tactile feedback needed in surgical training.

Therefore, it is an object of the invention to provide an affordable method for repetitive training of endoscopic work in a narrow channel, allowing training of the movements needed for resection of pituitary adenomas.

According to the invention, there is provided a **three-dimensional model** according to the teaching of claim 1.

Preferably, said pituitary gland portion of the model comprises a third portion having a third texture imitating the Sella turcica bottom to be removed/opened prior to accessing at least one of said first and second portions.

In a preferred embodiment, said first portion having a first texture comprises hard-boiled egg yolk, and said second portion having a second texture comprises hard-boiled egg white.

Preferably, said first portion having a first texture and said second portion having a second texture are constituted by a hard-boiled egg with its egg shell removed.

Alternatively, said first portion having a first texture, said second portion having a second texture and said third portion having a third texture may be constituted by a hard-boiled egg with its egg shell still in place.

Preferably, at least one of said nasal cavity portion and said pituitary gland portion of the model are individualized reconstructed portions based on three-dimensional image data of a particular person's head/skull.

More preferably, said individualized reconstructed portions have been reconstructed using an additive manufacturing method.

This additive manufacturing method may have been carried out using specific building materials and building material consolidation parameters for each of said first, second and third portions of the model having said first, second and third textures, respectively.

According to the invention, the model comprises a reusable main portion comprising a cavity for receiving at least one disposable sub portion comprising at least one of said nasal cavity portion and said pituitary gland portion of the model.

According to a second aspect of the invention, there is provided a **method for training** transnasal-access-type pituitary gland surgery using a three-dimensional training model as defined in any one of the preceding paragraphs, which method comprises the following steps:
a) inserting surgical instruments into a nasal cavity portion of the model and moving said surgical instruments along said nasal cavity portion from the outside of the model to said pituitary gland portion of the model;
c) resecting (removing) most or all of a first portion of said pituitary gland portion using said surgical instruments, said first portion having a first texture imitating unhealthy/abnormal optic nerve, brain and pituitary gland tissue;
d) not resecting (sparing) most or all of a second portion of said pituitary gland portion while using said surgical instruments, said second portion having a second texture imitating healthy/normal pituitary gland tissue.

Preferably, the method further comprises a step for
b) removing most or all of a third portion of said pituitary gland portion, said third portion having a third texture imitating the Sella turcica bottom, with step b) being carried out after step a) and before steps c) or d).

Most preferably, the surgical instruments comprise an endoscope or a microscope.

According to a third aspect of the invention, there is provided a **method for evaluating** the result of a method as defined in any one of the preceding three paragraphs, which method comprises the following steps:
e) measuring the amount of unresected material of said first portion of said pituitary gland portion, said first portion material having a first texture imitating abnormal pituitary gland tissue; and
f) measuring the amount of coresected material of said second portion of said pituitary gland portion at an interface between said first material and said second material, said second portion material having a second texture imitating normal pituitary gland tissue.

Preferably, the method further comprises a step for
g) measuring the amount of unresected material of said third portion of said pituitary gland portion, said third portion material having a third texture imitating the Sella turcica bottom; and
h) measuring the amount of coresected material of said second portion of said pituitary gland portion at an interface between said third material and said second material, said second portion material having a second texture imitating normal pituitary gland tissue.

### Brief description of the drawings

Fig. 1 is a perspective view of the three-dimensional training model according to the invention.
Fig. 2 shows the result of a training session where the method according to the invention has been carried out.

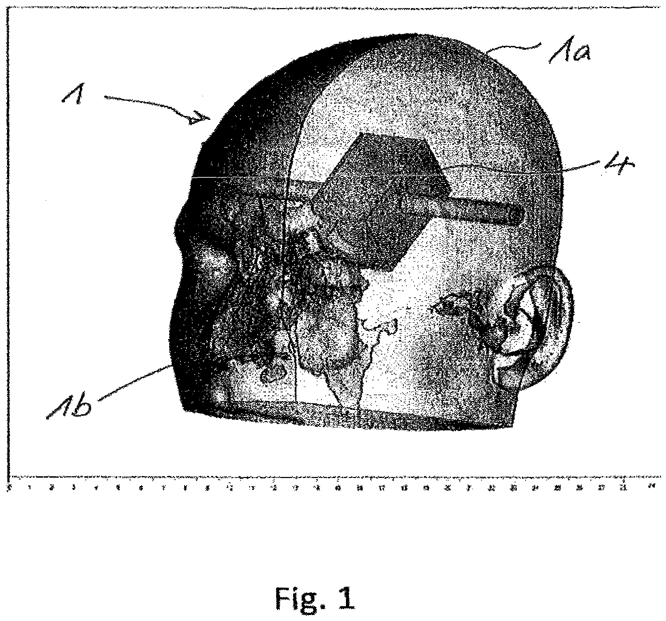

In Fig. 1, a perspective view of the three-dimensional training model 1 according to the invention is shown.

The model 1 has a reusable main portion 1a comprising a first cavity for receiving at first disposable sub portion 1b and a second cavity for receiving a second disposable sub portion 1c.

The first disposable sub portion 1b of the model 1 constitutes the nasal cavity portion 2 of a patient's head.

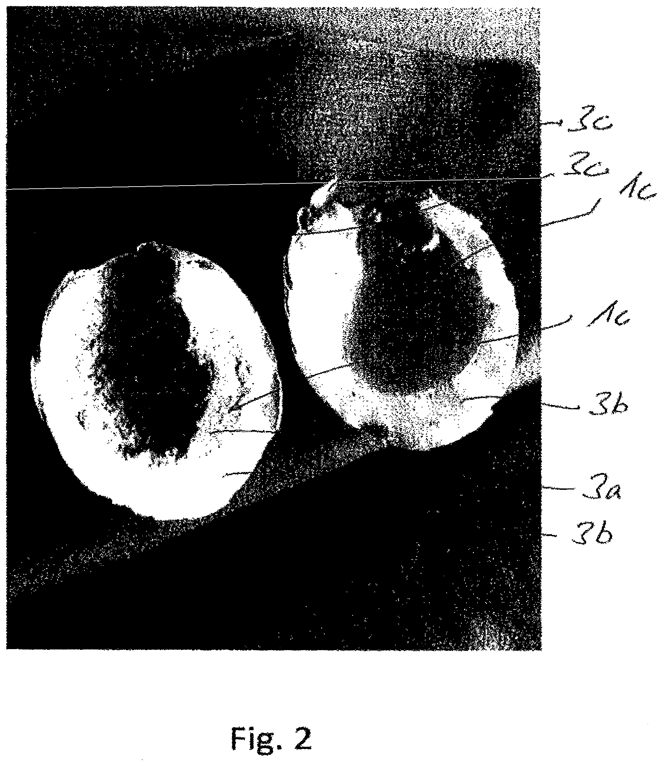

The second disposable sub portion 1c (Fig. 2) of the model 1 constitutes the pituitary gland portion 3 of a patient's head.

In a first version, a first portion 3a having a first texture and a second portion 3b having a second texture are constituted by a hard-boiled egg with its egg shell removed.

In a second version, the first portion 3a having a first texture, the second portion 3b having a second texture and a third portion 3c having a third texture are constituted by a hard-boiled egg with its egg shell still in place.

The hard-boiled egg with its shell removed or still in place is inserted and fixed in the box 4.

The nasal cavity portion 2 and/or the pituitary gland portion 3 of the model 1 may be individualized reconstructed portions based on three-dimensional image data of the patient's head/skull.

These individualized reconstructed portions 2, 3 have been reconstructed using an additive manufacturing method using specific building materials and building material consolidation parameters for each of the first, second and third portions of the model 1.

In Fig. 2, the result of a training session using the method according to the invention and hard-boiled eggs with their shell in place is shown. The hard-boiled eggs have been cut and only one half is shown.

In the hard-boiled egg on the left-hand side, only a small part of the egg yolk has been removed by a first trainee.

In the hard-boiled egg on the right-hand side, almost all the egg yolk has been removed by a second trainee and none of the egg white has been removed.

It can be seen that the second trainee did a much better job than the first trainee.

### Example

The final model 1 consists of a hard 3D reconstruction 1a of the head with a softer removable part 1b in the region of the nose and sphenoid sinus. The boiled egg (not shown) either with its shell removed or with its shell still in place is inserted in a dedicated box 4 at the level of the sella turcica.

The egg yolk constitutes the first portion 3a having a first texture imitating unhealthy/abnormal pituitary gland tissue to be resected.

The egg white constitutes the second portion 3b having a second texture imitating healthy/normal pituitary gland tissue not to be resected.

The egg shell constitutes the third portion 3c having a third texture imitating the sella turcica to be opened prior to accessing the first portion.

The model 1 allows training of the resection of the egg yolk under sparing of the egg white after careful opening of the shell. It is a realistic simulation of the movements done under the endoscope through a narrow approach at a depth of 12 cm.

### Conclusion

The inventive model and method achieve the goals mentioned above: they are affordable, offer tactile feedback and allow infinite repetitions. They could be used for training of advanced neurosurgical residents who thus far have very few possibilities of acquiring endoscopic experience.

## Claims

1. **Three-dimensional model** (1) of at least a portion of a human head or skull for training pituitary gland surgery by transnasal access, which model includes a nasal cavity portion (2) imitating the nasal cavity region of the head/skull and a pituitary gland portion (3) imitating the pituitary gland region of the head/skull, wherein said pituitary gland portion (3) can be accessed from the outside of the model (1) by surgical instruments extending along said nasal cavity portion (2) from the outside of the model (1) to said pituitary gland portion (3), wherein said pituitary gland portion (3) of the model comprises a first portion (3a) having a first texture imitating unhealthy/abnormal pituitary gland tissue to be resected, and a second portion (3b) having a second texture imitating healthy/normal pituitary gland tissue not to be resected **characterized in that** the model (1) comprises a reusable main portion (1a) comprising a cavity for receiving at least one disposable sub portion (1b, 1c) comprising at least one of said nasal cavity portion (2) and said pituitary gland portion (3) of the model (1).

2. Model (1) according to claim 1, **characterized in that** said pituitary gland portion (3) of the model (1) comprises a third portion (3c) having a third texture imitating the Sella turcica bottom to be removed/opened prior to accessing at least one of said first and second portions (3a, 3b).

3. Model (1) according to claim 1 or 2, **characterized in that** said first portion (3a) having a first texture comprises hard-boiled egg yolk, and said second portion (3b) having a second texture comprises hard-boiled egg white.

4. Model (1) according to claim 1, **characterized in that** said first portion (3a) having a first texture and said second portion (3b) having a second texture are constituted by a hard-boiled egg with its egg shell removed.

5. Model (1) according to claim 2, **characterized in that** said first portion (3a) having a first texture, said second portion (3b) having a second texture and said third portion (3c) having a third texture are constituted by a hard-boiled egg with its egg shell still in place.

6. Model (1) according to any one of claims 1 to 3, **characterized in that** at least one of said nasal cavity portion (2) and said pituitary gland portion (3) of the model are individualized reconstructed portions based on three-dimensional image data of a particular person's head/skull.

7. Model (1) according to claim 6, **characterized in that** said individualized reconstructed portions (2, 3) have been reconstructed using an additive manufacturing method.

8. Model (1) according to claim 7, **characterized in that** said additive manufacturing method has been carried out using specific building materials and building material consolidation parameters for each of said first, second and third portions of the model having said first, second and third textures, respectively.

9. **Method for training** transnasal-access-type pituitary gland surgery using a three-dimensional training model as defined in any one of claims 1 to 8, which method comprises the following steps:
a) inserting surgical instruments into a nasal cavity portion of the model and moving said surgical instruments along said nasal cavity portion from the outside of the model to said pituitary gland portion of the model;
c) resecting (removing) most or all of a first portion of said pituitary gland portion using said surgical instruments, said first portion having a first texture imitating unhealthy/abnormal pituitary gland tissue;
d) not resecting (sparing) most or all of a second portion of said pituitary gland portion while using said surgical instruments, said second portion having a second texture imitating healthy/normal pituitary gland tissue.

10. Method according to claim 9, **characterized by** a step for
b) removing most or all of a third portion of said pituitary gland portion, said third portion having a third texture imitating the Sella turcica bottom, with step b) being carried out after step a) and before steps c) or d).

11. Method according to claim 9 or 10, **characterized in that** said surgical instruments comprise an endoscope.

12. **Method for evaluating** the result of a method as defined in any one of claims 9 to 11, which method comprises the following steps:
e) measuring the amount of unresected material of said first portion of said pituitary gland portion, said first portion material having a first texture imitating abnormal pituitary gland tissue; and
f) measuring the amount of coresected material of said second portion of said pituitary gland portion at an interface between said first material and said second material, said second portion material having a second texture imitating normal pituitary gland tissue.

13. Method according to claim 12, **characterized by** a step for
g) measuring the amount of unresected material of said third portion of said pituitary gland portion, said third portion material having a third texture imitating the Sella turcica bottom; and
h) measuring the amount of coresected material of said second portion of said pituitary gland portion at an interface between said third material and said second material, said second portion material having a second texture imitating normal pituitary gland tissue.

## Patentansprüche

1. **Dreidimensionales Modell** (1) von zumindest einem Abschnitt eines menschlichen Kopfes oder Schädels zum Trainieren von Hirnanhangdrüsen-Chirurgie durch transnasalen Zugang, wobei das Modell einen den nasalen Kavitätsbereich des Kopfes/Schädels imitierenden nasalen Kavitätsabschnitt (2) und einen den Hirnanhangdrüsen-Bereich des Kopfes/Schädels imitierenden Hirnanhangdrüsen-Abschnitt (3) enthält, wobei der Zugang zu dem Hirnanhangdrüsen-Abschnitt (3) von der Aussenseite des Modells (1) durch chirurgische Instrumente erfolgen kann, die sich entlang des nasalen Kavitätsabschnitts (2) von der Aussenseite des Modells (1) zu dem Hirnanhangdrüsen-Abschnitt (3) erstrecken, wobei der Hirnanhangdrüsen-Abschnitt (3) des Modells einen ersten Abschnitt (3a) mit einer ersten Textur, welche zu resezierendes ungesundes/abnormales Hirnanhangdrüsen-Gewebe imitiert, und einen zweiten Abschnitt (3b) mit einer zweiten Textur, welche nicht zu resezierendes gesundes/normales Hirnanhangdrüsen-Gewebe imitiert, aufweist, **dadurch gekennzeichnet, dass** das Modell (1) einen wiederverwendbaren Hauptabschnitt (1a) aufweist mit einer Kavität zur Aufnahme mindestens eines wegwerfbaren Unterabschnitts (1b, 1c), welcher zumindest den nasalen Kavitätsabschnitt (2) und/oder den Hirnanhangdrüsen-Abschnitt (3) des Modells (1) aufweist.

2. Modell (1) nach Anspruch 1, **dadurch gekennzeichnet, dass** der Hirnanhangdrüsen-Abschnitt (3) des Modells (1) einen dritten Abschnitt (3c) mit einer dritten Textur aufweist, welche den Boden der Sella turcica imitiert, der zu entfernen/öffnen ist, bevor der Zugang zu mindestens dem ersten und/oder dem zweiten Abschnitt (3a, 3b) erfolgt.

3. Modell (1) nach Anspruch 1 oder 2, **dadurch gekennzeichnet, dass** der erste Abschnitt (3a) mit einer ersten Textur hartgekochten Eidotter aufweist, und der zweite Abschnitt (3b) mit einer zweiten Textur hartgekochtes Eiweiss aufweist.

4. Modell (1) nach Anspruch 1, **dadurch gekennzeichnet, dass** der erste Abschnitt (3a) mit einer ersten Textur und der zweite Abschnitt (3b) mit einer zweiten Textur durch ein hartgekochtes Ei gebildet sind, dessen Schale entfernt worden ist.

5. Modell (1) nach Anspruch 2, **dadurch gekennzeichnet, dass** der erste Abschnitt (3a) mit einer ersten Textur, der zweite Abschnitt (3b) mit einer zweiten Textur und der dritte Abschnitt (3c) mit einer dritten Textur durch ein hartgekochtes Ei gebildet sind, dessen Schale noch vorhanden ist.

6. Modell (1) nach einem der Ansprüche 1 bis 3, **dadurch gekennzeichnet, dass** zumindest der nasale Kavitätsabschnitt (2) und/oder der Hirnanhangdrüsen-Abschnitt (3) des Modells individualisierte rekonstruierte Abschnitte sind, welche auf dreidimensionalen Bilddaten des Kopfes/Schädels einer speziellen Person beruhen.

7. Modell (1) nach Anspruch 6, **dadurch gekennzeichnet, dass** die individualisierten rekonstruierten Abschnitte (2, 3) unter Verwendung eines additiven Fertigungsverfahrens rekonstruiert worden sind.

8. Modell (1) nach Anspruch 7, **dadurch gekennzeichnet, dass** das additive Fertigungsverfahren unter Verwendung spezifischer Baumaterialien und Baumaterial-Verfestigungsparameter jeweils für den ersten, den zweiten und den dritten Abschnitt des Modells mit der jeweils ersten, zweiten und dritten Textur durchgeführt worden ist.

9. **Verfahren zum Trainieren** von Transnasalzugang- Hirnanhangdrüsen -Chirurgie unter Verwendung eines dreidimensionalen Trainingsmodells gemäss einem der Ansprüche 1 bis 8, wobei das Verfahren die folgenden Schritte aufweist:
a) Einführen chirurgischer Instrumente in einen nasalen Kavitätsabschnitt des Modells und Bewegen der chirurgischen Instrumente entlang des nasalen Kavitätsabschnitts von der Aussenseite des Modells zu dem Hirnanhangdrüsen-Abschnitt des Modells;
c) Resezieren (Entfernen) eines Grossteils oder der Gesamtheit eines ersten Abschnitts des Hirnanhangdrüsen-Abschnitts unter Verwendung der chirurgischen Instrumente, wobei der erste Abschnitt eine erste Textur hat, welche ungesundes/abnormales Hirnanhangdrüsen-Gewebe imitiert;
d) Nicht-Resezieren (Schonen) eines Grossteils oder der Gesamtheit eines zweiten Abschnitts des Hirnanhangdrüsen-Abschnitts unter Verwendung der chirurgischen Instrumente, wobei der zweite Abschnitt eine zweite Textur hat, welche gesundes/normales Hirnanhangdrüsen-Gewebe imitiert.

10. Verfahren nach Anspruch 9, **gekennzeichnet durch** einen Schritt zum
b) Entfernen eines Grossteils oder der Gesamtheit eines dritten Abschnitts des Hirnanhangdrüsen-Abschnitts, wobei der dritte Abschnitt eine dritte Textur hat, welche den Boden der Sella turcica imitiert, wobei Schritt b) nach Schritt a) und vor den Schritten c) oder d) durchgeführt wird.

11. Verfahren nach Anspruch 9 oder 10, **dadurch gekennzeichnet, dass** die chirurgischen Instrumente ein Endoskop aufweisen.

12. **Verfahren zum Bewerten** des Ergebnisses eines Verfahrens gemäss einem der Ansprüche 9 bis 11, wobei das Verfahren die folgenden Schritte aufweist:
e) Messen der Menge von unreseziertem Material des ersten Abschnitts des Hirnanhangdrüsen-Abschnitts, wobei das Material des ersten Abschnitts eine erste Textur hat, welche abnormales Hirnanhangdrüsen-Gewebe imitiert; und
f) Messen der Menge von koreseziertem Material des zweiten Abschnitts des Hirnanhangdrüsen-Abschnitts an einer Grenzfläche zwischen dem ersten Material und dem zweiten Material, wobei das Material des zweiten Abschnitts eine zweite Textur hat, welche normales Hirnanhangdrüsen-Gewebe imitiert.

13. Verfahren nach Anspruch 12, **gekennzeichnet durch** einen Schritt zum
g) Messen der Menge von unreseziertem Material des dritten Abschnitts des Hirnanhangdrüsen-Abschnitts, wobei das Material des dritten Abschnitts eine dritte Textur hat, welche den Boden der Sella turcica imitiert; und
h) Messen der Menge von koreseziertem Material des zweiten Abschnitts des Hirnanhangdrüsen-Abschnitts an einer Grenzfläche zwischen dem dritten Material und dem zweiten Material, wobei das Material des zweiten Abschnitts eine zweite Textur hat, welche normales Hirnanhangdrüsen-Gewebe imitiert.

## Revendications

1. **Modèle tridimensionnel** (1) d'au moins une partie d'une tête ou d'un crâne humain pour l'entraînement de la chirurgie de l'hypophyse par accès transnasal, ledit modèle comprenant une partie de cavité nasale (2) imitant la région de cavité nasale de la tête / du crâne et une partie d'hypophyse (3) imitant la région d'hypophyse de la tête / du crâne, dans lequel ladite partie d'hypophyse (3) est accessible depuis l'extérieur du modèle (1) par des instruments chirurgicaux s'étendant le long de ladite partie de cavité nasale depuis l'extérieur du modèle (1) vers ladite partie d'hypophyse (3), dans lequel ladite partie d'hypophyse (3) du modèle comprend une première partie (3a) avec une première texture imitant du tissu malsain/anormal d'hypophyse qui doit être résectionné, et une deuxième partie (3b) avec une deuxième texture imitant du tissu sain/normal d'hypophyse qui ne doit pas être résectionné, **caractérisé en ce que** le modèle (1) comprend une partie principale réutilisable (1a) comprenant une cavité pour recevoir au moins une sous-partie jetable (1b, 1c) comprenant au moins une de ladite partie de cavité nasale (2) et de ladite partie d'hypophyse (3) du modèle (1).

2. Modèle (1) selon la revendication 1, **caractérisé en ce que** ladite partie d'hypophyse (3) du modèle (1) comprend une troisième partie (3c) avec une troisième texture imitant le fond de la selle turcique (sella turcica) qui doit être enlevée/ouverte avant l'accès à au moins une desdites première et deuxième parties (3a, 3b).

3. Modèle (1) selon la revendication 1 ou 2, **caractérisé en ce que** ladite première partie (3a) avec une première texture comprend du jaune d'oeuf dur, et ladite deuxième partie (3b) avec une deuxième texture comprend du blanc d'oeuf dur.

4. Modèle (1) selon la revendication 1, **caractérisé en ce que** ladite première partie (3a) avec une première texture et ladite seconde partie (3b) avec une deuxième texture sont constituées par un oeuf dur dont la coquille a été enlevée.

5. Modèle (1) selon la revendication 2, **caractérisé en ce que** ladite première partie (3a) avec une première texture, ladite seconde partie (3b) avec une deuxième texture et ladite troisième partie (3c) avec une troisième texture sont constituées par un oeuf dur dont la coquille est toujours en place.

6. Modèle (1) selon l'une quelconque des revendications 1 à 3, **caractérisé en ce qu'**au moins une de ladite partie de cavité nasale (2) et de ladite partie d'hypophyse (3) du modèle sont des parties individualisées reconstruites qui sont basées sur des données tridimensionelles d'image de la tête / du crâne d'une personne particulière.

7. Modèle (1) selon la revendication 6, **caractérisé en ce que** lesdites parties individualisées reconstruites (2, 3) ont été reconstruites en utilisant un procédé de fabrication additive.

8. Modèle (1) selon la revendication 7, **caractérisé en ce que** ledit procédé de fabrication additive a été réalisé en utilisant des matériaux de construction et des paramètres de consolidation spécifiques pour chacune desdites première, deuxième et troisième parties du modèle avec respectivement ladite première, deuxième et troisième texture.

9. **Méthode d'entraînement** de la chirurgie d'hypophyse du type transnasal utilisant un modèle tridimensionnel d'entraînement selon l'une des revendications 1 à 8, ladite méthode comprenant des étapes consistant à:
a) insérer des instruments chirurgicaux dans une partie de cavité nasale du modèle et déplacer lesdits instruments chirurgicaux le long de ladite partie de cavité nasale depuis l'extérieur du modèle vers ladite partie d'hypophyse du modèle;
c) résectionner (enlever) la plupart ou la totalité d'une première partie de ladite partie d'hypophyse en utilisant lesdits instruments chirurgicaux, ladite première partie ayant une première texture imitant du tissu malsain/anormal d'hypophyse;
d) ne pas résectionner (épargner) la plupart ou la totalité d'une deuxième partie de ladite partie d'hypophyse en utilisant lesdits instruments chirurgicaux, ladite deuxième partie ayant une deuxième texture imitant du tissu sain/normal d'hypophyse.

10. Méthode selon la revendication 9, **caractérisée par** une étape consistant à
b) enlever la plupart ou la totalité d'une troisième partie de ladite partie d'hypophyse, ladite troisième partie ayant une troisième texture imitant le fond de la selle turcique (sella turcica), l'étape b) étant réalisée après l'étape a) et avant les étapes c) ou d).

11. Méthode selon la revendication 9 ou 10, **caractérisée en ce que** lesdits instruments chirurgicaux comprennent un endoscope.

12. **Méthode d'évaluation** du résultat d'une méthode selon l'une des revendications 9 à 11, ladite méthode comprenant des étapes consistant à:
e) mesurer la quantité de matière non-résectionnée de ladite première partie de ladite partie d'hypophyse, ladite matière de première partie ayant une première texture imitant du tissu anormal d'hypophyse; et
f) mesurer la quantité de matière corésectionnée de ladite deuxième partie de ladite partie d'hypophyse à une interface entre ladite première matière et ladite deuxième matière, ladite matière de deuxième partie ayant une deuxième texture imitant du tissu normal d'hypophyse.

13. Méthode selon la revendication 12, **caractérisée par** une étape consistant à
g) mesurer la quantité de matière non-résectionnée de ladite troisième partie de ladite partie d'hypophyse, ladite matière de troisième partie ayant une troisième texture imitant le fond de la selle turcique (sella turcica); et
h) mesurer la quantité de matière corésectionnée de ladite deuxième partie de ladite partie d'hypophyse à une interface entre ladite troisième matière et ladite deuxième matière, ladite matière de deuxième partie ayant une deuxième texture imitant du tissu normal d'hypophyse.
